# EUROPÄISCHE PATENTSCHRIFT

(11) **EP 3 513 760 B1**
(45) Veröffentlichungstag und Bekanntmachung des Hinweises auf die Patenterteilung: **27.05.2020**
(21) Anmeldenummer: 19152956.9
(22) Anmeldetag: 22.01.2019
(51) Int. Cl.: A61B 34/00, G16H 40/20, G06F 3/0354, G06F 3/038, G16H 40/67, A61B 17/00

(54) **SYSTEM ZUR STEUERUNG EINES MEDIZINISCHEN GERÄTS**
SYSTEM FOR CONTROLLING A MEDICAL DEVICE
SYSTÈME DE COMMANDE D'UN APPAREIL MÉDICAL

(30) Priorität: 22.01.2018 DE 102018101345
(43) Veröffentlichungstag der Anmeldung: 24.07.2019
(73) Patentinhaber: Karl Storz SE & Co. KG, 78532 Tuttlingen (DE)
(72) Erfinder: Brüderle, Klaus, 78532 Tuttlingen (DE); Zeller, Marco, 78532 Tuttlingen (DE); Rüppell, Jan, 78532 Tuttlingen (DE); Baumann, Harald, 78532 Tuttlingen (DE)
(74) Vertreter: Witte, Weller & Partner Patentanwälte mbB

(56) Entgegenhaltungen:
- WO-A1-2017/025644
- DE-A1-102011 083 957
- DE-A1-102014 208 463
- US-A1- 2013 096 575

## Beschreibung

Die vorliegende Erfindung betrifft ein System zur Steuerung eines medizinischen Geräts.

In einem Operationssaal befinden sich eine Vielzahl von medizinischen Geräten, die während einer Operation eingesetzt werden, wobei oftmals mehrere medizinische Geräte gleichzeitig verwendet werden. Die medizinischen Geräte verfügen in aller Regel über ein Bedienfeld, mit dem die Geräte gesteuert und gewünschte Einstellungen vorgenommen werden können. Da das Bedienfeld am entsprechenden medizinischen Gerät fest und unveränderlich angeordnet ist, wird mit einem bestimmten Bedienfeld stets auch nur ein bestimmtes medizinisches Gerät gesteuert.

Aufgrund der Vielzahl der medizinischen Geräte, die während einer Operation eingesetzt werden, ist es nicht möglich, alle medizinischen Geräte in unmittelbarer Nähe des Benutzers anzuordnen. Vielmehr werden medizinische Geräte gerade in solchen Bereichen angeordnet, in denen sie den Ärzten oder dem Assistenzpersonal während einer Operation nicht im Weg sind. Bevorzugt befinden sich die medizinischen Geräte in einer Entfernung vom zentralen Operationstisch, um freie Bewegungsabläufe zu ermöglichen. Dies führt nun aber dazu, dass eine Bedienung vieler medizinischer Geräte nur noch möglich ist, indem sich der Benutzer zum medizinischen Gerät hin bewegt.

Um diesem Problem zu begegnen, haben Fernbedienungen im Operationssaal Einzug gehalten. Diese Fernbedienungen ermöglichen es, ein medizinisches Gerät aus der Ferne zu bedienen und daran Einstellungen vorzunehmen. Wenngleich Fernbedienungen wesentlich kompakter sind als das medizinische Gerät selbst, ist es für einen Benutzer jedoch schwierig, während einer Operation mehrere Fernbedienungen auseinanderzuhalten und stets, insbesondere in einer Notfallsituation, die richtige Fernbedienung für das benötigte medizinische Gerät zu verwenden.

Hinsichtlich dieses Problems haben Bildschirme mit variabler Darstellung, insbesondere berührungsempfindliche Bildschirme wie Touchscreens oder Tablets, eine erhebliche Erleichterung geschaffen. Es besteht nun die Möglichkeit, Daten von verschiedenen medizinischen Geräten auf dem Bildschirm darzustellen und, im Falle eines berührungsempfindlichen Bildschirms, auch Befehle für verschiedene medizinische Geräte entgegenzunehmen. Dabei können auf dem Bildschirm entweder Bedienelemente für mehrere Geräte nebeneinander oder nacheinander angezeigt werden. Die verschiedenen Darstellungen können vom Benutzer manuell ausgewählt werden oder automatisch umgeschaltet werden. Somit kann ein Benutzer nun auf einfache Weise mehrere medizinische Geräte fernbedienen ohne sich in unmittelbarer Nähe dieser Geräte befinden zu müssen.

Die Möglichkeit, eine Vielzahl von medizinischen Geräten von verschiedenen Personen über verschiedene Fernbedienungen, insbesondere Tablets, aus der Ferne bedienen zu können, ist sehr attraktiv. Dabei muss jedoch sichergestellt werden, dass zu keinem Zeitpunkt ein fehlerhafter Befehl an ein medizinisches Gerät gesendet und dort ausgeführt wird. Eine Erleichterung beider Bedienung der medizinischen Geräte darf nicht zu erhöhten Risiken bei einem Patienten führen. US2013096575 offenbart ein System zur Gestensteuerung eines medizinischen Geräts.

Es ist eine Aufgabe der vorliegenden Erfindung ein System zur Steuerung eines medizinischen Geräts aufzuzeigen, bei dem eine Ausführung von fehlerhaften Bedienbefehlen durch das medizinische Gerät verhindert werden kann.

Die Erfindung wird im angehängten unabhängigen Anspruch 1 definiert, bevorzugte Ausführungsformen werden in den abhängigen Ansprüchen beschrieben.

Die Aufgabe wird gelöst durch ein System mit
- einem ersten Kanal und einem zweiten Kanal,
- einer Schnittstelle zum Empfangen von Anzeigedaten und Kontrolldaten, die dafür ausgebildet ist, die Anzeigedaten und Kontrolldaten in den ersten Kanal zu senden und die Kontrolldaten in den zweiten Kanal zu senden,
- wobei der erste Kanal aufweist:
   - einen Bildgeber, der dafür ausgebildet ist, die Anzeigedaten und die Kontrolldaten als Bild auf dem Bildgeber anzuzeigen,
   - einen Bildaufnehmer aufweist, der dafür ausgebildet ist, zumindest den auf dem Bildgeber dargestellten Bildteil des Bilds, der mit den Kontrolldaten korrespondiert, als Bilddaten zu erfassen, und
   - eine Extrahiereinrichtung, die dafür ausgebildet ist, aus den Bilddaten Prüfdaten zu extrahieren,
- wobei der zweite Kanal eine Steuereinrichtung aufweist, die dafür ausgebildet ist, Eingaben eines Benutzers entgegenzunehmen, die der Steuerung des medizinischen Geräts dienen, und im Fall einer Eingabe die Kontrolldaten und einen Steuerbefehl auszugeben, ansonsten die Kontrolldaten auszugeben,
- und mit einer Vergleichseinrichtung, die dafür ausgebildet ist, die Prüfdaten von der Extrahiereinrichtung und die Kontrolldaten von der Steuereinrichtung zu vergleichen, ein Vergleichsergebnis zu ermitteln und das Vergleichsergebnis auszugeben.

Im Rahmen der Erfindung haben die Erfinder erkannt, dass für eine zuverlässige und sichere Kommunikation zwischen einer Fernbedienung, insbesondere einem Tablet, und einem medizinischen Gerät verschiedene Aspekte überprüft werden sollten. Zum einen sollte ein Befehl nur ausgeführt werden, wenn er durch einen berechtigten Benutzer abgesetzt wurde. Der Befehl soll aus dem Kontext der aktuellen Prozessinformation hervorgehen. Das Senden eines Befehls und zugehöriger Information soll überwacht und validiert werden. Schließlich sollen der Befehlsfluss und der zugehörige Informationsfluss sicher sein und hinsichtlich ihrer Integrität überprüft werden.

Es handelt sich bei dem System um ein zweikanaliges System mit einem ersten Kanal und einem zweiten Kanal. Das System verfügt über eine Schnittstelle, die Anzeigedaten und Kontrolldaten empfängt. Bei den Anzeigedaten handelt es sich um Informationen, die zur Anzeige auf dem Bildgeber gebracht werden. Dies schließt alle Elemente ein, die auf dem Bildgeber dargestellt werden können, einschließlich numerischer Werte, Textdarstellungen, graphischen Darstellungen und Bedienelemente, wie zum Beispiel Tasten oder Regler. Die Anzeigedaten sind dafür ausgebildet, auf dem Bildgeber eine für den Benutzer verständliche Information betreffend die Funktion des medizinischen Geräts darzustellen.

Es sei darauf hingewiesen, dass der erste und der zweite Kanal nicht physikalisch getrennt sein müssen. Vielmehr wird es als vorteilhaft angesehen, wenn die Kanäle physikalisch nicht getrennt sind, bevorzugt sich einen physikalischen Kanal teilen. Insbesondere soll jeder der Kanäle als virtueller Signalpfad verstanden werden, welcher die Informationspakete zwischen den Komponenten transportiert. Die Kanäle sind aber dennoch logisch voneinander getrennt, d.h. die in ihnen enthaltenen Informationen sind isoliert, insbesondere in Nachrichten gekapselt. Diese Nachrichten können bevorzugt in Paketen zusammengefasst sein und über eine gemeinsame physikalische Datenübertragungseinrichtung oder Netzwerk in einem Rahmen übertragen werden. Selbst wenn mehrere Komponenten an dieselbe physikalische Verbindung angeschlossen sind, erfolgt die Trennung der Kanäle dann insbesondere dadurch, dass die Komponenten entweder nur solche Pakete erhalten oder nur auf solche Pakete reagieren, z.B. auswerten, die für die jeweilige Komponente bestimmt sind.

Bei den Kontrolldaten handelt es sich um Daten, die eine Unterscheidung zwischen verschiedenen Datenpaketen ermöglicht. Die Kontrolldaten können zwar unter Umständen von einem Benutzer wahrgenommen werden, sind jedoch nicht für eine Interpretation für den Benutzer ausgelegt, sondern dienen der sicheren Steuerung, die nachfolgend noch erläutert wird.

Die Schnittstelle sendet zumindest die empfangenen Anzeigedaten und Kontrolldaten in den ersten Kanal und sendet die Kontrolldaten in den zweiten Kanal.

Im ersten Kanal gelangen die Anzeigedaten und Kontrolldaten zum Bildgeber, auf dem diese Daten als Bild angezeigt werden. Der Bildaufnehmer erfasst zumindest den Bildteil des Bilds als Bilddaten, auf dem die Kontrolldaten dargestellt werden. Eine Extrahiereinrichtung extrahiert dann aus den Bilddaten sog. Prüfdaten. Die Prüfdaten korrespondieren dabei mit den Kontrolldaten und sind idealerweise, wenn also kein Fehler vorliegt, identisch zu den Kontrolldaten. Eine Abweichung hingegen gibt einen Hinweis auf einen Fehler.

Dabei wird für das Extrahieren bevorzugt ein Verfahren angewendet, das die Wandlung der Kontrolldaten in auf dem Bildgeber dargestellte Informationen umkehrt. Werden die Kontrolldaten auf dem Bildgeber zum Beispiel als Barcode dargestellt, so extrahiert die Extrahiereinrichtung aus den Bilddaten den darin enthaltenen Barcode, um die Prüfdaten zu erhalten. Werden die Kontrolldaten bspw. als alphanumerische Zeichen dargestellt, so extrahiert die Extrahiereinrichtung die Prüfdaten aus den Bilddaten mittels optischer Zeichenerkennung (OCR).

Der zweite Kanal, in den die Kontrolldaten gesendet werden, weist eine Steuereinrichtung auf. Diese nimmt Eingaben eines Benutzers entgegen, der das medizinische Gerät steuern möchte. Wenn die Steuereinrichtung die Kontrolldaten erhält und der Benutzer eine Eingabe vornimmt oder seit dem letzten Empfangen von Kontrolldaten eine Eingabe vorgenommen hat, werden die Kontrolldaten zusammen mit einem Steuerbefehl ausgegeben, der die vom Benutzer gewünschte Steuerung des medizinischen Geräts repräsentiert. Hat der Benutzer keine Eingabe vorgenommen, so wird kein Steuerbefehl ausgegeben.

In einer nachgeschalteten Vergleichseinrichtung werden die Prüfdaten von der Extrahiereinrichtung und die Kontrolldaten von der Steuereinrichtung verglichen und es wird ein entsprechendes Vergleichsergebnis ermittelt. Das Vergleichsergebnis enthält bevorzugt die Information, ob eine Übereinstimmung besteht oder ob keine Übereinstimmung besteht. In Abhängigkeit von der Ausgestaltung der Kontrolldaten kann alternativ oder zusätzlich ein numerischer Unterschied zwischen den Prüfdaten und den Kontrolldaten als Vergleichsergebnis ermittelt werden. Das Vergleichsergebnis wird dann für eine weitere Verarbeitung ausgegeben. Ist das Vergleichsergebnis positiv, d.h. die Prüfdaten stimmen mit den Kontrolldaten überein, wird von einer ordnungsgemäßen Funktion des Systems ausgegangen. Ist das Vergleichsergebnis negativ, d.h. die Prüfdaten unterscheiden sich von den Kontrolldaten, wird von einem Fehler innerhalb des Systems ausgegangen. Das System kann bei bevorzugten Ausführungsformen im Fall eines negativen Vergleichsergebnisses ein Signal ausgeben, z.B. ein optisches Signal, ein akustisches Signal oder ein Datensignal. Der Vergleich oder Schritt des Vergleichens kann basierend auf einer Vielzahl von Darstellungen der Kontrolldaten durchgeführt werden, einschließlich, aber nicht beschränkt auf, eines oder mehrere aus der Gruppe von Bildern, Texten, Farben, Formen, Buchstaben, Zahlen, alphanumerischen Daten, Zeichen, Barcodes oder Symbolen. Wenn beispielsweise die Kontrolldaten als erstes Bild dargestellt werden, wird das zweite Bild, das die Testdaten darstellt, mit dem ersten Bild unter Verwendung bekannter Algorithmen zum Bildvergleich verglichen. Wenn die Kontrolldaten alphanumerische Daten sind und das zweite Bild Daten enthält, die als Bild kodiert sind, z.B. ein Barcode, können diese Daten aus dem zweiten Bild extrahiert und dann mit den alphanumerischen Daten der Steuerdaten verglichen werden. Bei einigen vorteilhaften Ausführungsformen muss durch den Vergleich eine vollständige Übereinstimmung gefunden werden, um ein positives Ergebnis zu erzielen. Bei anderen vorteilhaften Ausführungsformen wird ein Schwellwert, z.B. ein fester oder variabler Prozentsatz, verwendet, und wenn die Übereinstimmung der Prüfdaten mit den Kontrolldaten diesen Schwellenwert überschreitet, führt der Vergleich zu einem positiven Ergebnis.

Wenngleich das System viele offensichtliche Vorteile gegenüber dem Stand der Technik bietet, soll auf eine Besonderheit ausdrücklich hingewiesen werden. Wie oben erläutert, werden die Anzeigedaten zusammen mit den Kontrolldaten auf dem Bildgeber ausgegeben. Stimmen die Prüfdaten bei dem späteren Vergleich mit den Kontrolldaten überein, so ist einerseits bekannt, dass aktuelle Kontrolldaten auf dem Bildgeber angezeigt werden. Aufgrund der gleichzeitigen Darstellung bedeutet dies aber auch, dass auf dem Bildgeber aktuelle Anzeigedaten angezeigt werden. Wäre die Darstellung auf dem Bildgeber eingefroren, so würde der Benutzer dies eventuell nicht bemerken und könnte auf der Basis von veralteten Anzeigedaten einen Steuerbefehl senden. Eine solche Situation kann nun erkannt werden, da aufgrund der eingefrorenen Anzeige die Prüfdaten nicht mehr mit den Kontrolldaten übereinstimmen.

Das System macht sich die Annahme zunutze, dass eine erste Laufzeit im ersten Kanal, also vom Empfang an der Schnittstelle bis zum Erreichen der Vergleichseinrichtung, gleich der Laufzeit im zweiten Kanal ist oder ein Laufzeitunterschied zwischen dem ersten Kanal und dem zweiten Kanal entweder bekannt oder bestimmbar ist. Ein eventueller Laufzeitunterschied kann dann kompensiert werden. So wird sichergestellt, dass eine Abweichung zwischen den Kontrolldaten und den Prüfdaten nicht bereits durch einen Laufzeitunterschied hervorgerufen wird.

Dadurch ist die Aufgabe vollständig gelöst.

Bei einer vorteilhaften Ausgestaltung ist der Bildgeber ein Bildschirm, insbesondere ein berührungsempfindlicher Bildschirm, und/oder ist der Bildaufnehmer eine Kamera.

Es handelt sich bei diesen Elementen um weitverbreitete Produkte, die einen hohen Reifegrad haben und am Markt in vielen Varianten erhältlich sind. Dabei ist es insbesondere bevorzugt, wenn der Bildgeber ein Tablet ist, da ein Tablet neben einer sehr guten Bildschirmanzeige sowohl über fortgeschrittene Möglichkeit der Befehlseingabe als auch drahtlose Kommunikationsschnittstellen, wie zum Beispiel WLAN oder Bluetooth verfügt.

Bei einer weiteren vorteilhaften Ausgestaltung ist der Bildgeber dafür ausgebildet, die Kontrolldaten in einem vorbestimmten Bereich des Bilds darzustellen und die Extrahiereinrichtung ist dafür ausgebildet, die Prüfdaten aus dem vorbestimmten Bereich zu extrahieren.

Grundsätzlich ist es möglich, die auf dem Bildgeber dargestellten Kontrolldaten zu extrahieren, sofern der Bereich, in dem die Kontrolldaten angezeigt werden, vom Bildaufnehmer erfasst wird. So ist es bspw. möglich, den vollständigen Bildschirm des Bildgebers mittels des Bildaufnehmers zu erfassen, auch wenn die Kontrolldaten nur in einem kleinen Teil der Anzeige dargestellt sind. Es wird aber als vorteilhaft angesehen, wenn die Kontrolldaten in einem vorbestimmten Bereich des Bilds dargestellt werden. Der Bildaufnehmer kann dann gezielt den vorbestimmten Bereich auswerten. Dies macht die Auswertung und Gewinnung der Prüfdaten besonders effektiv und zuverlässig.

Bei einer weiteren vorteilhaften Ausgestaltung ist der Bildgeber dafür ausgebildet, die Kontrolldaten mit einem vorbestimmten Kennzeichen darzustellen und die Extrahiereinrichtung ist dafür ausgebildet, die Prüfdaten aus einem durch das vorbestimmte Kennzeichen gekennzeichneten Bereich zu extrahieren.

Diese Ausgestaltung ermöglicht es, dass die Extrahiereinrichtung die in dem Bild dargestellten Kontrolldaten schnell identifizieren kann und die Prüfdaten somit gezielt extrahieren kann. Als Kennzeichen können insbesondere solche graphischen Elemente verwendet werden, die sich hinsichtlich ihrer Form und/oder ihrer Anordnung von anderen auf dem Bildgeber dargestellten Elementen unterscheiden. Beispielsweise kann ein 2D-Barcode verwendet werden.

Bei einer weiteren vorteilhaften Ausgestaltung weist das System ferner eine Bildauswerteeinrichtung auf, die dafür ausgebildet ist, eine Anwesenheit eines Gesichts in den Bilddaten zu prüfen, ein Anwesenheitsergebnis zu ermitteln und das Anwesenheitsergebnis auszugeben.

Bei dieser Ausgestaltung wird angenommen, dass der Bildaufnehmer zusätzlich zu der Erfassung des Bildteils mit den Kontrolldaten auch einen Bereich abbildet, in dem ein Gesicht des Benutzers, der Eingaben mit der Steuereinrichtung vornimmt, zu erkennen sein müsste. Auf diese Weise kann überprüft werden, ob sich vor dem Bildgeber eine Person befindet, die die Anzeige auf dem Bildgeber betrachtet. Auf diese Weise kann zusätzlich überprüft werden, ob der Benutzer von der Darstellung auf dem Bildgeber Notiz nimmt. Zusätzlich oder alternativ kann bei einer Bedienung der Steuereinrichtung überprüft werden, ob dies in Anwesenheit eines Benutzers erfolgt ist. So kann eine versehentliche Bedienung der Steuereinrichtung erkannt werden.

Bei einer weiteren vorteilhaften Ausgestaltung ist die Bildauswerteeinrichtung ferner dafür ausgebildet, in einem anwesenden Gesicht einen Lidschlag zu identifizieren und eine solche Identifikation beim Ermitteln des Anwesenheitsergebnisses zu berücksichtigen.

Diese Ausgestaltung bietet eine zusätzliche Sicherheit bei der Überprüfung, ob tatsächlich ein Benutzer vor dem Bildgeber anwesend ist. So wird insbesondere zunächst ein Gesicht in den Bilddaten identifiziert, dann ein Augenbereich in dem Gesicht identifiziert und dann innerhalb des Augenbereichs eine deutliche Veränderung, insbesondere ein Lidschlag identifiziert. Werden ein Gesicht und ein Lidschlag identifiziert, kann mit hoher Wahrscheinlichkeit ausgeschlossen werden, dass ein Gesicht irrtümlich erkannt wurde oder dass ein Manipulationsversuch vorliegt, zum Beispiel durch ein ausgedrucktes Bild von einem Gesicht.

Bei einer weiteren vorteilhaften Ausgestaltung ist die Bildauswerteeinrichtung ferner dafür ausgebildet, eine Bewegung eines anwesenden Gesichts zu identifizieren und eine solche Identifikation beim Ermitteln des Anwesenheitsergebnisses zu berücksichtigen.

Auch diese Ausgestaltung erhöht die Zuverlässigkeit bei der Feststellung, ob ein Benutzer vor dem Bildgeber anwesend ist. Ein positives Anwesenheitsergebnis wird nur dann ausgegeben, wenn sowohl ein Gesicht identifiziert wurde als auch eine Bewegung des identifizierten Gesichts festgestellt wurde. Bei einer weiteren vorteilhaften Ausgestaltung wird ein positives Anwesenheitsergebnis nur dann ausgegeben, wenn ein Gesicht identifiziert wurde, ein Lidschlag in dem Gesicht identifiziert wurde und eine Bewegung des Gesichts identifiziert wurde.

Bei einer weiteren vorteilhaften Ausgestaltung weisen die Kontrolldaten einen Zeitstempel auf, der beim Ermitteln des Vergleichsergebnisses dahingehend berücksichtigt wird, dass ein negatives Vergleichsergebnis ermittelt wird, wenn der Zeitstempel von einer aktuellen Zeit um mehr als eine vorbestimmte Zeitdauer abweicht.

Diese Ausgestaltung ermöglicht eine weitere Überprüfung, ob das System ordnungsgemäß arbeitet. Es ist bspw. denkbar, dass sowohl im ersten Kanal als auch im zweiten Kanal eine Zeitverzögerung auftritt. Zwar stimmen dann möglicherweise die Kontrolldaten und die Prüfdaten überein, doch deutet der Zeitversatz auf ein mögliches Problem hin. Bei Erkennung dieses möglichen Problems, kann dann eine Überprüfung oder eine Fehlerbehandlung eingeleitet werden. Bei dem Zeitstempel handelt es sich bevorzugt um die aktuelle Zeit zum Zeitpunkt der Erzeugung der Kontrolldaten.

Bei einer weiteren vorteilhaften Ausgestaltung weisen die Kontrolldaten eine Sequenzposition auf, die beim Ermitteln des Vergleichsergebnisses dahingehend berücksichtigt wird, dass ein negatives Vergleichsergebnis ermittelt wird, wenn die Sequenzposition nicht zu einer erwarteten Sequenzposition passt.

Bei dieser Ausgestaltung werden die Kontrolldaten mit einer Sequenzposition gesendet. Dem liegt die Annahme zugrunde, dass Kontrolldaten, die in einer bestimmten Reihenfolge von der Sendeeinrichtung empfangen werden auch in einer entsprechenden Reihenfolge an der Vergleichseinrichtung ankommen sollen. Wird anhand der Sequenzposition der aktuell vorliegenden Prüfdaten und/oder Kontrolldaten festgestellt, dass diese nicht zu der vorherigen Sequenzposition oder zu den vorherigen Sequenzpositionen passt, wird ein negatives Vergleichsergebnis ausgegeben.

Bei einer weiteren vorteilhaften Ausgestaltung ist der Bildgeber dafür ausgebildet, eine Geste zu detektieren, die von einem Benutzer mit mindestens einem Finger auf dem Bildgeber ausgeführt wird und weist das System ferner eine Bildauswerteeinrichtung auf, die dafür ausgebildet ist, zumindest den Beginn oder das Ende derselben Geste mittels des Bildaufnehmers zu detektieren.

Bei dieser Ausgestaltung wird davon ausgegangen, dass der Steuerbefehl mittels einer Geste auf dem Bildgeber ausgeführt wird. Um zu überprüfen, ob die vom Bildgeber, insbesondere einem berührungsempfindlichen Bildschirm oder Tablet, detektiert wurde, auch tatsächlich mittels eines Fingers des Benutzers ausgeführt wurde. Zu diesem Zweck wird ein Bild oder mehrere Bilder, das/die vom Bildaufnehmer aufgenommen wurde/wurden von der Bildauswerteeinrichtung ausgewertet. Wird eine räumliche und/oder zeitliche Übereinstimmung hinsichtlich der erkannten Geste festgestellt, so besteht eine hohe Zuverlässigkeit dahingehend, dass die detektierte Geste tatsächlich von einem Benutzer ausgeführt wurde.

Bei einer weiteren vorteilhaften Ausgestaltung ist der Bildaufnehmer ortsfest zum Bildgeber angeordnet.

Diese Ausgestaltung ist relativ einfach zu realisieren. Es ist zwar grundsätzlich auch möglich, dass der Bildaufnehmer im Raum angeordnet ist und von dort das auf dem Bildgeber dargestellte Bild aufzeichnet. Das Extrahieren der auf den Bildgeber dargestellten Kontrolldaten wird aber dann besonders einfach, wenn der Bildaufnehmer ortsfest zum Bildgeber angeordnet ist. Dabei können, je nach gewünschter Ausführungsform, sowohl das auf dem Bildgeber angezeigte Bild als auch ein Bereich vor dem Bildgeber zur Erfassung eines Gesichts aufgezeichnet werden.

Bei einer weiteren vorteilhaften Ausgestaltung weist das System ferner eine Freigabeeinheit auf, die dafür ausgebildet ist, eine Ausführung des Steuerbefehls durch ein medizinisches Gerät zuzulassen, falls das Vergleichsergebnis positiv ist, und zu unterdrücken, falls das Vergleichsergebnis negativ ist.

Bei dieser Ausgestaltung kann zusätzlich oder alternativ zu der genannten Signalfunktion die Ausführung des Steuerbefehls unterdrückt werden, wenn es einen Hinweis auf eine Fehlfunktion gibt. Die Unterdrückung des Steuerbefehls kann bspw. dadurch erfolgen, dass der Steuerbefehl nicht an das medizinische Gerät gesendet wird oder dass der Steuerbefehl vom medizinischen Gerät nicht ausgeführt wird. Wenn für die Ausführung eines Steuerbefehls die Anwesenheit eines Benutzers vor dem Bildgeber erforderlich ist, kann auch das Anwesenheitsergebnis berücksichtigt werden. Eine Ausführung des Steuerbefehls durch das medizinische Gerät wird nur dann zugelassen, wenn sowohl das Vergleichsergebnis als auch das Anwesenheitsergebnis positiv ist. Die Ausführung wird unterdrückt, falls entweder das Vergleichsergebnis oder das Anwesenheitsergebnis negativ ist. Eine solche Überprüfung kann sowohl außerhalb des medizinischen Geräts, zum Beispiel in einer Steuereinheit, oder im medizinischen Gerät selbst erfolgen.

Bei einer weiteren vorteilhaften Ausgestaltung ist die Freigabeeinheit ferner dafür ausgebildet, die Ausführung des Steuerbefehls zu unterdrücken, falls der Befehl nicht mit dem medizinischen Gerät korrespondiert, dessen Daten und/oder Bedienelemente in dem Bild auf dem Bildgeber angezeigt sind.

Auf diese Weise kann verhindert werden, dass ein Steuerbefehl trotz positivem Vergleichsergebnis und positivem Anwesenheitsergebnis ausgeführt wird, wenn die erforderliche Übereinstimmung nicht besteht. Hierzu kann bspw. in den Kontrolldaten eine entsprechende Codierung übermittelt werden, die entweder vor dem Senden des Steuerbefehls an ein medizinisches Gerät oder vom medizinischen Gerät selbst überprüft wird. Gelangt bspw. ein Abschaltbefehl an ein erstes medizinisches Gerät, obwohl zum Zeitpunkt der Erzeugung des Steuerbefehls eine Oberfläche für ein zweites medizinisches Gerät angezeigt wurde, so wird die Ausführung des Steuerbefehls unterdrückt.

Bei einer weiteren vorteilhaften Ausgestaltung der Erfindung werden die Kontrolldaten im medizinischen Gerät erzeugt und wird das Vergleichsergebnis im medizinischen Gerät ermittelt.

Diese Ausgestaltung stellt sicher, dass die Überwachung und die Überprüfung an der Stelle stattfindet, wo letztendlich die Steuerbefehle ausgeführt werden und somit auch ein fehlerhafter Steuerbefehl ausgeführt werden könnte. Dadurch kann weitestgehend ausgeschlossen werden, dass durch nachfolgende Fehlerquellen doch noch ein fehlerhafter Steuerbefehl ausgeführt wird.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung näher dargestellt und werden in der nachfolgenden Beschreibung näher erläutert. Es zeigen:
- Fig. 1: eine Ausführungsform eines oben beschriebenen Systems;
- Fig. 2: eine Ausführungsform eines Bildgebers in einem dem oben beschriebenen System; und
- Fig. 3: die Ausführungsform gemäß Fig. 2 im Zusammenspiel mit einem medizinischen Gerät.

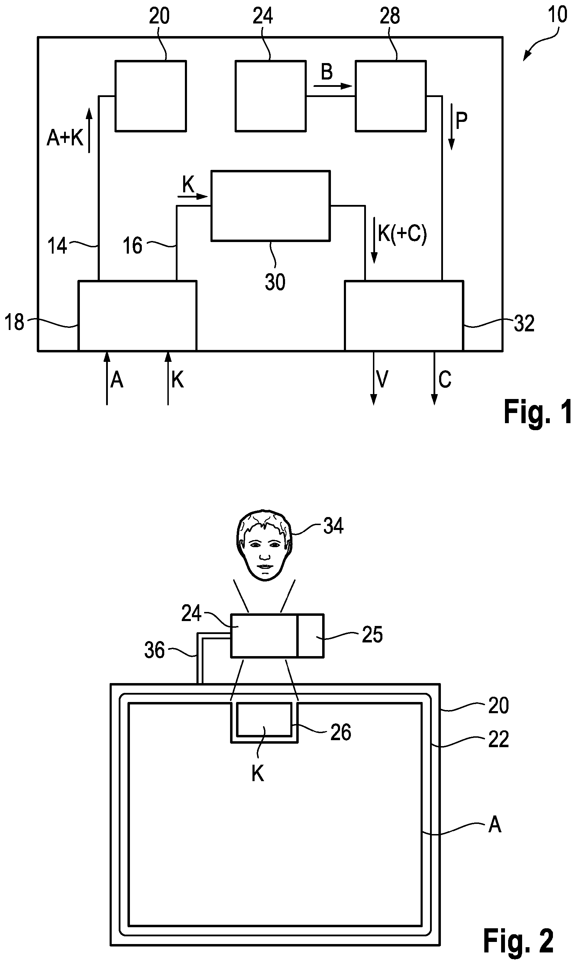

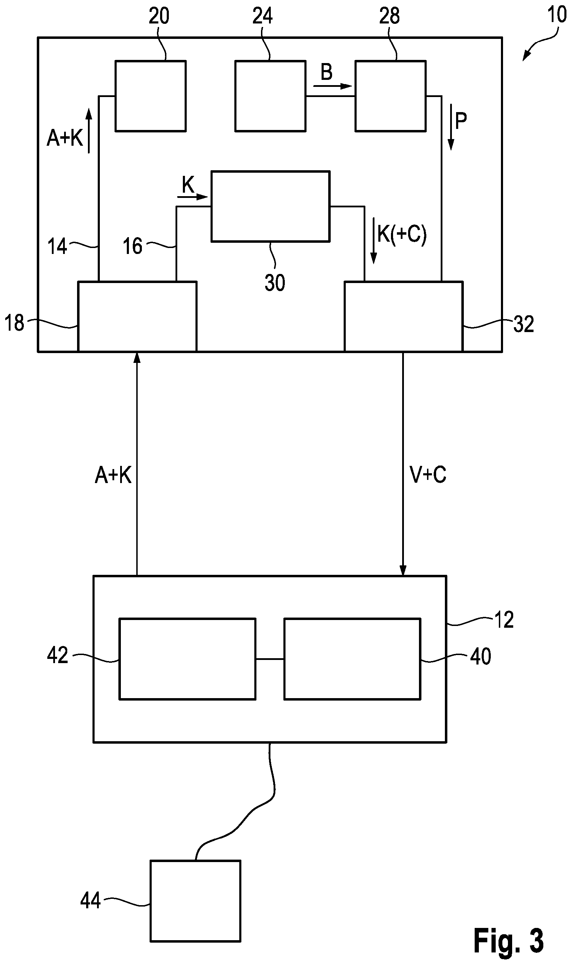

Fig. 1 zeigt ein System 10 zur Steuerung eines medizinischen Geräts 12 (siehe Fig. 3). Das System weist einen ersten Kanal 14 und einen zweiten Kanal 16 auf. Das System 10 hat ferner eine Schnittstelle 18 zum Empfangen von Anzeigedaten A und Kontrolldaten K, wobei die Schnittstelle 18 dafür ausgebildet ist, die Anzeigedaten A und die Kontrolldaten K in den ersten Kanal 14 zu senden und die Kontrolldaten K in den zweiten Kanal 16 zu senden.

Der erste Kanal 14 weist einen Bildgeber 20 auf, insbesondere einen Bildschirm, der dafür ausgebildet ist, die Anzeigedaten A und die Kontrolldaten K als Bild 22 (siehe Fig. 2) auf den Bildgeber 20 anzuzeigen. Der erste Kanal 14 weist ferner einen Bildaufnehmer 24 auf, der dafür ausgebildet ist, zumindest den auf dem Bildgeber 20 dargestellten Bildteil 26 (siehe Fig. 2) des Bilds 22, der mit den Kontrolldaten K korrespondiert, als Bilddaten B zu erfassen. Der erste Kanal 14 weist zudem eine Extrahiereinrichtung 28 auf, die dafür ausgebildet ist, aus den Bilddaten B Prüfdaten P zu extrahieren.

Der zweite Kanal 16 weist eine Steuereinrichtung 30 auf, die dafür ausgebildet ist, Eingaben eines Benutzers entgegenzunehmen, die der Steuerung des medizinischen Geräts 12 dienen, und im Fall einer Eingabe die Kontrolldaten K und einen Steuerbefehl C auszugeben, ansonsten die Kontrolldaten K auszugeben. Das System 10 weist schließlich auch eine Vergleichseinrichtung 32 auf, die dafür ausgebildet ist, die Prüfdaten P von der Extrahiereinrichtung 28 und die Kontrolldaten K von der Steuereinrichtung 30 zu vergleichen, ein Vergleichsergebnis V zu ermitteln und das Vergleichsergebnis V auszugeben.

Durch die Pfeile ist angedeutet, dass die Anzeigedaten A und die Kontrolldaten K der Schnittstelle 18 zugeführt werden. Ferner ist dargestellt, dass aus der Vergleichseinrichtung 32 das Vergleichsergebnis V und der Steuerbefehl C herausgeführt werden. Das Vergleichsergebnis V und der Steuerbefehl C können dann einer Freigabeeinheit 40 (siehe Fig. 3) zugeführt werden. Es ist aber auch möglich, die Freigabeeinheit 40 in die Vergleichseinrichtung 32 zu integrieren, so dass ein Steuerbefehl C nur bei einem positiven Vergleichsergebnis V nach außen gesendet wird, d.h. an das medizinische Gerät 12 gesendet wird.

Fig. 2 zeigt den Bildgeber 20, der das Bild 22 darstellt. Dabei sind in dem Bildteil 26 die Kontrolldaten K dargestellt und im verbleibenden Bereich des Bilds 22 die Anzeigedaten A. Ein Bildaufnehmer 24, hier eine Kamera, erfasst dabei zum einen die Kontrolldaten K sowie ein vor dem Bildgeber 20 befindliches Gesicht 34. Mittels eines symbolischen Halters 36 ist angedeutet, dass der Bildaufnehmer 24 ortsfest zum Bildgeber 20 angeordnet ist.

Fig. 3 zeigt das System 10 gemäß Fig. 1 im Zusammenspiel mit dem medizinischen Gerät 12. Das medizinische Gerät 12 weist neben der Freigabeeinheit 40 auch eine Gerätsteuerung 42 auf. Bei dem hier gezeigten Beispiel wird mittels des medizinischen Geräts 12 ein Insufflator 44 gesteuert. Während des Betriebs erzeugt das medizinische Gerät 12 Anzeigedaten A und Kontrolldaten K. Dabei werden die Anzeigedaten A von der Gerätsteuerung 42 erzeugt und die Kontrolldaten K von der Freigabeeinheit 40 oder von der Gerätsteuerung 42. Die Anzeigedaten A und die Kontrolldaten K werden an die Schnittstelle 18 gegeben und durchlaufen wie oben beschrieben den ersten Kanal 14 und den zweiten Kanal 16. Die Vergleichseinrichtung 32 sendet dann das Vergleichsergebnis V, hier zusammen mit dem Steuerbefehl C, zurück an das medizinische Gerät 12. Bei einem positiven Vergleichsergebnis V führt das medizinische Gerät 12 den Steuerbefehl C aus. Dies bedeutet in aller Regel, dass eine Einstellung am Insufflator 44 geändert wird. Falls das Vergleichsergebnis V negativ ist, unterdrückt die Freigabeeinheit 40 die Ausführung des Steuerbefehls C.

Bei einer anderen Ausführungsform werden die Prüfdaten P aus dem ersten Kanal 14, die Kontrolldaten K aus dem zweiten Kanal 16 und der Steuerbefehl C zum medizinischen Gerät 12 gesendet. Das Vergleichsergebnis V wird dann im medizinischen Gerät 12 ermittelt. Eine solche Ausgestaltung hat den Vorteil, dass innerhalb des medizinischen Geräts 12 zusätzlich geprüft werden kann, ob die gesendeten Kontrolldaten K mit den empfangenen Prüfdaten P und den empfangenen Kontrolldaten K übereinstimmen. Die Vergleichseinrichtung 32 ist dann bevorzugt im medizinischen Gerät 12 angeordnet. Die Freigabeeinheit 40 kann ferner prüfen, ob der Steuerbefehl C mit den Anzeigedaten A korrespondiert.

## Patentansprüche

1. System (10) zur Steuerung eines medizinischen Geräts (12), das System (10) mit
- einem ersten Kanal (14) und einem zweiten Kanal (16),
- einer Schnittstelle (18) zum Empfangen von Anzeigedaten (A) und Kontrolldaten (K), die dafür ausgebildet ist, die Anzeigedaten (A) und Kontrolldaten (K) in den ersten Kanal (14) zu senden und die Kontrolldaten (K) in den zweiten Kanal (16) zu senden,
- wobei der erste Kanal (14) aufweist:
- einen Bildgeber (20), der dafür ausgebildet ist, die Anzeigedaten (A) und die Kontrolldaten (K) als Bild (22) auf dem Bildgeber (20) anzuzeigen,
- einen Bildaufnehmer (24) aufweist, der dafür ausgebildet ist, zumindest den auf dem Bildgeber (20) dargestellten Bildteil (26) des Bilds (22), der mit den Kontrolldaten (K) korrespondiert, als Bilddaten (B) zu erfassen, und
- eine Extrahiereinrichtung (28), die dafür ausgebildet ist, aus den Bilddaten (B) Prüfdaten (P) zu extrahieren,
- wobei der zweite Kanal (16) eine Steuereinrichtung (30) aufweist, die dafür ausgebildet ist, Eingaben eines Benutzers entgegenzunehmen, die der Steuerung des medizinischen Geräts (12) dienen, und im Fall einer Eingabe die Kontrolldaten (K) und einen Steuerbefehl (C) auszugeben, ansonsten die Kontrolldaten (K) auszugeben,
- und mit einer Vergleichseinrichtung (32), die dafür ausgebildet ist, die Prüfdaten (P) von der Extrahiereinrichtung (28) und die Kontrolldaten (K) von der Steuereinrichtung (30) zu vergleichen, ein Vergleichsergebnis (V) zu ermitteln und das Vergleichsergebnis (V) auszugeben.

2. System nach Anspruch 1, wobei der Bildgeber (20) ein Bildschirm ist, insbesondere ein berührungsempfindlicher Bildschirm, und/oder der Bildaufnehmer (24) eine Kamera ist.

3. System nach einem der vorhergehenden Ansprüche, wobei der Bildgeber (20) dafür ausgebildet ist, die Kontrolldaten (K) in einem vorbestimmten Bereich des Bilds (22) darzustellen und die Extrahiereinrichtung (28) dafür ausgebildet ist, die Prüfdaten (P) aus dem vorbestimmten Bereich zu extrahieren.

4. System nach einem der vorhergehenden Ansprüche, wobei der Bildgeber (20) dafür ausgebildet ist, die Kontrolldaten (K) mit einem vorbestimmten Kennzeichen darzustellen und die Extrahiereinrichtung (28) dafür ausgebildet ist, die Prüfdaten (P) aus einem durch das vorbestimmte Kennzeichen gekennzeichneten Bereich zu extrahieren.

5. System nach einem der vorhergehenden Ansprüche, wobei das System (10) ferner eine Bildauswerteeinrichtung (25) aufweist, die dafür ausgebildet ist, eine Anwesenheit eines Gesichts (34) in den Bilddaten (B) zu prüfen, ein Anwesenheitsergebnis zu ermitteln und das Anwesenheitsergebnis auszugeben.

6. System nach Anspruch 5, wobei die Bildauswerteeinrichtung (25) ferner dafür ausgebildet ist, in einem anwesenden Gesicht (34) einen Lidschlag zu identifizieren und eine solche Identifikation beim Ermitteln des Anwesenheitsergebnisses zu berücksichtigen.

7. System nach Anspruch 5 oder 6, wobei die Bildauswerteeinrichtung (25) ferner dafür ausgebildet ist, eine Bewegung eines anwesenden Gesichts (34) zu identifizieren und eine solche Identifikation beim Ermitteln des Anwesenheitsergebnisses zu berücksichtigen.

8. System nach einem der vorhergehenden Ansprüche, wobei die Kontrolldaten (K) einen Zeitstempel aufweisen, der beim Ermitteln des Vergleichsergebnisses (V) dahingehend berücksichtigt wird, dass ein negatives Vergleichsergebnis (V) ermittelt wird, wenn der Zeitstempel von einer aktuellen Zeit um mehr als eine vorbestimmte Zeitdauer abweicht.

9. System nach einem der vorhergehenden Ansprüche, wobei die Kontrolldaten (K) eine Sequenzposition aufweisen, die beim Ermitteln des Vergleichsergebnisses (V) dahingehend berücksichtigt wird, dass ein negatives Vergleichsergebnis (V) ermittelt wird, wenn die Sequenzposition nicht zu einer erwarteten Sequenzposition passt.

10. System nach einem der vorhergehenden Ansprüche, wobei der Bildgeber (20) dafür ausgebildet ist eine Geste zu detektieren, die von einem Benutzer mit mindestens einem Finger auf dem Bildgeber (20) ausgeführt wird, und wobei das System (10) ferner eine Bildauswerteeinrichtung (25) aufweist, die dafür ausgebildet ist, zumindest den Beginn oder das Ende derselben Geste mittels des Bildaufnehmers (24) zu detektieren.

11. System nach einem der vorhergehenden Ansprüche, wobei der Bildaufnehmer (24) ortsfest zum Bildgeber (20) angeordnet ist.

12. System nach einem der vorhergehenden Ansprüche, wobei das System (10) ferner eine Freigabeeinheit (40) aufweist, die dafür ausgebildet ist, eine Ausführung des Steuerbefehls (C) durch ein medizinisches Gerät (12) zuzulassen, falls das Vergleichsergebnis (V) positiv ist, und zu unterdrücken, falls das Vergleichsergebnis (V) negativ ist.

13. System nach Anspruch 12, wobei die Freigabeeinheit (40) ferner dafür ausgebildet ist, die Ausführung des Steuerbefehls (C) zu unterdrücken, falls der Befehl nicht mit dem medizinischen Gerät (12) korrespondiert, dessen Daten und/oder Bedienelemente in dem Bild (22) auf dem Bildgeber (20) angezeigt sind.

14. System nach einem der vorhergehenden Ansprüche, wobei die Kontrolldaten (K) im medizinischen Gerät (12) erzeugt wird und das Vergleichsergebnis (V) im medizinischen Gerät (12) ermittelt wird.

## Claims

1. System (10) for controlling a medical device (12), the system (10) comprising
- a first channel (14) and a second channel (16),
- an interface (18) for receiving display data (A) and control data (K), the interface (18) adapted to transmit the display data (A) and control data (K) into the first channel (14) and to transmit the control data (K) into the second channel (16),
- wherein the first channel (14) has
- an imager (20) adapted to display the display data (A) and the control data (K) as an image (22) on the imager (20),
- has an image sensor (24) which is designed to detect as image data (B) at least the image part (26) of the image (22) displayed on the image generator (20) which corresponds to the control data (K), and
- extracting means (28) adapted to extract test data (P) from the image data (B),
- wherein the second channel (16) comprises a control device (30) adapted to receive inputs from a user for controlling the medical device (12) and to output the control data (K) and a control command (C) in case of an input, otherwise to output the control data (K),
- and with a comparison device (32) which is designed to compare the test data (P) from the extraction device (28) and the control data (K) from the control device (30), to determine a comparison result (V) and to output the comparison result (V).

2. System according to claim 1, wherein the image generator (20) is a screen, in particular a touch-sensitive screen, and/or the image sensor (24) is a camera.

3. System according to any of the preceding claims, wherein the imager (20) is adapted to display the control data (K) in a predetermined area of the image (22) and the extracting means (28) is adapted to extract the control data (P) from the predetermined area.

4. System according to any of the preceding claims, wherein the imager (20) is adapted to display the control data (K) with a predetermined characteristic and the extracting means (28) is adapted to extract the test data (P) from a region marked by the predetermined characteristic.

5. System according to any of the preceding claims, the system (10) further comprising an image evaluating device (25) adapted to check a presence of a face (34) in the image data (B), to determine a presence result and to output the presence result.

6. System according to claim 5, wherein the image evaluation device (25) is further adapted to identify an eyelid blink in a face (34) present and to take such identification into account when determining the presence result.

7. System according to claim 5 or 6, wherein the image evaluation device (25) is further adapted to identify a movement of a face (34) present and to take such identification into account when determining the presence result.

8. System according to one of the preceding claims, wherein the control data (K) has a time stamp which is taken into account in determining the comparison result (V) in that a negative comparison result (V) is determined if the time stamp deviates from a current time by more than a predetermined period of time.

9. System according to any of the preceding claims, wherein the control data (K) has a sequence position which is taken into account when determining the comparison result (V) in that a negative comparison result (V) is determined if the sequence position does not match an expected sequence position.

10. System according to any of the preceding claims, wherein the imager (20) is adapted to detect a gesture performed by a user with at least one finger on the imager (20), and wherein the system (10) further comprises an image evaluation device (25) adapted to detect at least the beginning or the end of the same gesture by means of the imager (24).

11. System according to one of the preceding claims, wherein the image sensor (24) is arranged stationary relative to the image generator (20).

12. System according to any of the preceding claims, the system (10) further comprising a release unit (40) adapted to permit execution of the control command (C) by a medical device (12) if the comparison result (V) is positive and to suppress it if the comparison result (V) is negative.

13. System according to claim 12, wherein the enabling unit (40) is further adapted to suppress the execution of the control command (C) if the command does not correspond to the medical device (12) whose data and/or operating elements are displayed in the image (22) on the imager (20).

14. System according to one of the preceding claims, wherein the control data (K) is generated in the medical device (12) and the comparison result (V) is determined in the medical device (12).

## Revendications

1. Système (10) de commande d'un appareil médical (12), le système (10) comprenant
- un premier canal (14) et un second canal (16),
- une interface (18) pour la réception de données d'affichage (A) et de données de contrôle (K), qui est conçue pour transmettre les données d'affichage (A) et les données de contrôle (K) dans le premier canal (14) et pour transmettre les données de contrôle (K) dans le second canal (16),
- dans lequel le premier canal (14) comporte :
- un générateur d'image (20) qui est conçu pour afficher les données d'affichage (A) et les données de contrôle (K) sous la forme d'une image (22) sur le générateur d'image (20),
- un capteur d'image (24) qui est conçu pour détecter, en tant que données d'image (B), au moins la partie d'image (26) de l'image (22) représentée sur le générateur d'image (20), qui correspond aux données de contrôle (K), et
- un dispositif d'extraction (28) qui est conçu pour extraire des données de test (P) des données d'image (B),
- dans lequel le second canal (16) comprend un dispositif de commande (30) qui est conçu pour recueillir des saisies d'un utilisateur, lesquelles servent à commander l'appareil médical (12), et pour délivrer les données de contrôle (K) et un ordre de commande (C) dans le cas d'une saisie, et sinon pour délivrer les données de contrôle (K),
- et un dispositif de comparaison (32) qui est conçu pour comparer les données de test (P) du dispositif d'extraction (28) et les données de contrôle (K) du dispositif de commande (30), pour déterminer un résultat de comparaison (V) et pour délivrer le résultat de comparaison (V).

2. Système selon la revendication 1, dans lequel le générateur d'image (20) est un écran, en particulier un écran tactile, et/ou le capteur d'image (24) est une caméra.

3. Système selon l'une des revendications précédentes, dans lequel le générateur d'image (20) est conçu pour représenter les données de contrôle (K) dans une zone prédéterminée de l'image (22) et le dispositif d'extraction (28) est conçu pour extraire les données de test (P) de la zone prédéterminée.

4. Système selon l'une des revendications précédentes, dans lequel le générateur d'image (20) est conçu pour afficher les données de contrôle (K) avec une caractéristique prédéterminée, et le dispositif d'extraction (28) est conçu pour extraire les données de test (P) d'une région identifiée par la caractéristique prédéterminée.

5. Système selon l'une des revendications précédentes, dans lequel le système (10) comporte en outre un dispositif d'évaluation d'image (25) qui est conçu pour tester la présence d'un visage (34) dans les données d'image (B), pour déterminer un résultat de présence et pour délivrer le résultat de présence.

6. Système selon la revendication 5, dans lequel le dispositif d'évaluation d'image (25) est en outre conçu pour identifier un clignement dans un visage (34) présent et pour prendre en compte cette identification lors de la détermination du résultat de présence.

7. Système selon la revendication 5 ou 6, dans lequel le dispositif d'évaluation d'image (25) est en outre conçu pour identifier un mouvement d'un visage (34) présent et pour prendre en compte cette identification lors de la détermination du résultat de présence.

8. Système selon l'une des revendications précédentes, dans lequel les données de contrôle (K) comportent un marqueur temporel qui est pris en compte lors de la détermination du résultat de comparaison (V) en faisant en sorte qu'un résultat de comparaison négatif (V) soit déterminé lorsque le marqueur temporel diffère du temps actuel de plus d'une durée prédéterminée.

9. Système selon l'une des revendications précédentes, dans lequel les données de contrôle (K) présentent une position séquentielle qui est prise en compte lors de la détermination du résultat de comparaison (V) en faisant en sorte qu'un résultat de comparaison négatif (V) soit déterminé lorsque la position séquentielle ne correspond pas à une position séquentielle attendue.

10. Système selon l'une des revendications précédentes, dans lequel le générateur d'image (20) est conçu pour détecter un geste effectué par un utilisateur avec au moins un doigt sur le générateur d'image (20), et dans lequel le système (10) comporte en outre un dispositif d'évaluation d'image (25) qui est conçu pour détecter au moins le début ou la fin d'un même geste au moyen du capteur d'image (24).

11. Système selon l'une des revendications précédentes, dans lequel le capteur d'image (24) est disposé de manière fixe par rapport au générateur d'image (20).

12. Système selon l'une des revendications précédentes, dans lequel le système (10) comprend en outre une unité de déclenchement (40) qui est conçue pour permettre l'exécution de l'ordre de commande (C) par un appareil médical (12) dans le cas où le résultat de comparaison (V) est positif et pour l'empêcher dans le cas où le résultat de comparaison (V) est négatif.

13. Système selon la revendication 12, dans lequel l'unité de déclenchement (40) est en outre conçue pour empêcher l'exécution de l'ordre de commande (C) dans le cas où l'ordre ne correspond pas à l'appareil médical (12) dont les données et/ou les éléments de commande sont affichés dans l'image (22) sur le générateur d'image (20).

14. Système selon l'une des revendications précédentes, dans lequel les données de contrôle (K) sont générées dans l'appareil médical (12) et le résultat de la comparaison (V) est déterminé dans l'appareil médical (12).
